# EUROPÄISCHE PATENTSCHRIFT

(11) **EP 1 287 831 B1**
(45) Veröffentlichungstag und Bekanntmachung des Hinweises auf die Patenterteilung: **22.11.2006**
(21) Anmeldenummer: 01121105.9
(22) Anmeldetag: 03.09.2001
(51) Int. Cl.: A61K 39/00, C07K 7/06, A61K 39/385, A61P 35/00

(54) **Antigen-Mimotope und Vakzine gegen Krebserkrankungen**
Antigen mimotopes and vaccine against cancerous diseases
Mimotopes d'antigènes et vaccin contre des maladies cancéreuses

(43) Veröffentlichungstag der Anmeldung: 05.03.2003
(73) Patentinhaber: Bio Life Science Forschungs- und Entwicklungsges.m.b.H., 1010 Wien (AT)
(72) Erfinder: Zielinski, Christoph, 1180 Wien (AT); Scheiner, Otto, 2380 Perchtoldsdorf (AT); Jensen-Jarolim, Erika, 1210 Wien (AT); Breiteneder, Heimo, 1070 Wien (AT); Pehamberger, Hubert, 1230 Wien (AT)
(74) Vertreter: Kador & Partner

(56) Entgegenhaltungen:
- WO-A-00/38515
- WO-A-01/26672
- WO-A-89/11296
- S. FERRONE ET AL.: "Active specific immunotherapy of malignant melanoma and peptide mimics of the human high molecular weight melanoma associated antigen." RECENT RESULTS IN CANCER RESEARCH, Bd. 158, 2001, Seiten 231-235, XP001053502 Berlin, Deutschland
- X. WANG ET AL.: "Immunotherapy of melanoma: peptide mimics of a human high molecular weight melanoma associated antigen." MEDICINA, Bd. 60, Nr. suppl. 2, August 2000 (2000-08), Seiten 48-50, XP001053496 Buenos Aires, Argentinien
- B. SPONER ET AL.: "Mimotopes for the induction of a humoral immune response to the high molecular weight-melanoma associated antigen. Application of phage display." JOURNAL OF INVESTIGATIVE DERMATOLOGY, Bd. 114, April 2000 (2000-04), Seite 850 XP001009896 New York, NY, VSA
- E. NORONHA ET AL.: "Mimicry of the human high molecular weight-melanoma associated antigen (HMW-MAA) by peptides isolated with human and mouse antibodies." PROCEEDINGS OF THE AMERICAN ASSOCIATION FOR CANCER RESEARCH, Bd. 40, März 1999 (1999-03), Seite 357 XP002189953 VSA
- M. GEISER ET AL.: "Identification of the human melanoma-associated chondroitin sulfate proteoglycan antigen epitope recognized by the antitumor monoclonal antibody 763.74 from a peptide phage library." CANCER RESEARCH, Bd. 59, 15. Februar 1999 (1999-02-15), Seiten 905-910, XP002189954 Philadelphia, PA, VSA

## Beschreibung

Die vorliegende Erfindung betrifft eine Vakzine gegen Krebserkrankungen sowie Antigen-Mimotope, die mit dem High Molecular Weight Melanoma Associated Antigen (HMW-MAA) assoziiert sind.

In den letzten Jahren war weltweit ein stetiger Zuwachs an Melanomfällen zu verzeichnen. Das Melanom ist der Tumor mit der höchsten Steigerungsrate, die in Mitteleuropa und in den USA mit 5 % pro Jahr angegeben wird. Die Inzidenz beträgt derzeit in Mitteleuropa etwa 12 - 15 je 100 000 Einwohnern jährlich und hat sich innerhalb der letzten 10 Jahre verdoppelt, wobei es auch weltweit zu einer Zunahme der Erkrankungsfälle kam. Die weltweit höchsten Inzidenzen werden aus Australien und den Südstaaten der USA mit ca. 30 Fällen je 100 000 Einwohnern pro Jahr berichtet. Das Melanom tritt in allen Altersstufen auf, vor dem 15. Lebensjahr ist es ein seltenes Ereignis. Das durchschnittliche Manifestationsalter liegt bei 56 Jahren. Das Melanom ist definitionsgemäß ein maligner Tumor der Melanozyten. Die maligne Proliferation äußert sich in einer horizontalen Wachstumsphase bzw. in einer vertikalen Wachstumsphase mit Knotenbildung.

Eine wichtige Rolle beim Tumorwachstum und bei der Zelladhäsion spielt das an über 90 % der Melanome exprimierte High Molecular Weight Melanoma Associated Antigen (HMW-MAA) oder das MCSP-Melanoma Associated Chondroitin Sulfate Proteoglycan. Das HMW-MAA besteht aus einer großen extrazellulären Domäne mit 2222 Aminosäuren, einem kleineren 25 Aminosäuren großen transmembranen Segment sowie einer cytoplasmatischen Domäne bestehend aus 75 Aminosäuren (EMBL accession number X96753). Wegen seiner hohen Expressionsrate an Melanomzellen und aufgrund seiner hohen Immunogenität sind bereits eine Reihe von monoklonalen Mausantikörpern produziert worden, welche in unterschiedlicher Weise klinisch eingesetzt werden.

Das HMW-MAA ist eine effektive Zielstruktur für radiodiagnostische Fragestellungen und seine monoklonalen anti-idiotypischen Antikörper finden bei der spezifischen Immuntheraphie des Melanoms Verwendung. Auf diese Weise konnte in einer Studie eine humorale anti HMW-MAA Immunität in ungefähr 60 % der immunisierten Patienten mit fortgeschrittenem Melanom induziert werden (Mittelman A., Chen Z.J., Yang H., Wong G.Y., Ferrone S., Proc. Natl. Acad. Sci. USA, **1992** Jan 15, 89 (2), 466-70). Weiterhin wurde festgestellt, dass diese humorale Immunität mit einer statistisch signifikanten Verlängerung der Überlebensrate vergesellschaftet war (Mittelman A., Chen Z.J., Wong G.Y., Liu C., Hirai S., Ferrone S., Clin. Cancer Res. **1995** July 1, 7, 705-13).

Einer der oben erwähnten monoklonalen Antikörper ist der monoklonale HMW-MAA Antikörper 225.28S. Dieser Antikörper ist spezifisch für das HMW-MAA und besitzt im Vergleich zu anderen Antikörpern ein spezielles Paratop, welches an ein Epitop der extrazellulären Domäne des HMW-MAA bindet (Ziai M.R., Imberti L., Nicotra M.R., Badaracco G., Segatto O., Natali P.G., Ferrone S., Cancer Res., **1987** May 1, 47 (9), 2474-80).

Gegen den HMW-MAA Antikörper 225.28S wurden bereits anti-idiotypische Antikörper entwickelt. Diese anti-idiotypischen Antikörper MELIMMUNE1 und MF11-30 wurden in klinischen Studien zur spezifischen Immuntherapie eingesetzt und zeigten neben der Induktion einer humoralen Immunantwort (Mittelman A., Chen Z.J., Kageshita T., Yang H., Yamada M., Baskind P., Goldberg N., Puccio C., Ahmed T., Arlin Z., Ferrone S., J. Clin. Invest. **1990** Dec., 86, 2136-2144), auch eine Induktion von spezifischen CTLs (cytotoxische T-Lymphocyten) gegen Tumorzellen (Pride M.W., Shuey S., Grillo-Lopez A., Braslawsky G., Ross M., Legha S.S., Eton O., Buzaid A., Ioannides C., Murray J.L., Clin. Cancer Res. **1998** Oct., 4(10), 2363-70).

Obwohl die auf anti-idiotypische Antikörper erhaltene Immunantwort ein wünschenswertes Ergebnis darstellt, können die damit verbundenen klinischen Probleme wie etwa die Induktion von humanen Antikörpern gegen Mausimmunglobulin G (HAMA) nicht unberücksichtigt bleiben.

Aus diesem Grund wurde versucht, die anti-idiotypischen Antikörper mittels Mimotope zu ersetzen. Beispielsweise wurde von Geiser et al (Geiser M., Schultz D., Le Cardinal A., Voshol H., Garcia-Echeverria C., Cancer Res., **1999**, Feb. 15, 59 (4), 905-10) mittels einer Peptidphagenbibliothek das Antigenepitop des menschlichen melanom-assoziierten Chontroitinsulfatproteoglycan identifiziert. Weiterhin wurde von Ferrone et al (Ferrone S., Wang X., Recent Results Cancer Res., **2001**, 158, 231-5) ein aus einer Phagenpeptidbibliothek mittels des 225.28S Antikörpers erhaltenes 15-meres Peptid publiziert, welches eine gewisse Homologie zu der extrazellulären Domäne des HMW-MAA aufweist. WO 01/26672 bezieht sich auf eine Vakzine gegen Krebserkrankungen, die mit HMW-MAA assoziiert sind.

Es ist demgemäß Aufgabe der vorliegenden Erfindung, eine Vakzine gegen Krebserkrankungen oder ein Antigen-Mimotop, die mit dem High Molecular Weight Melanoma Associated Antigen (HMW-MAA) assoziiert sind, bereitzustellen, mit deren Hilfe es möglich ist, die Nachteile herkömmlicher Krebsbehandlungsmethoden zu vermeiden, eine wirksame Prophylaxe von solchen Krebserkrankungen zu ermöglichen, sowie ein Mittel zur Verfügung zu stellen, mit welchem solche Krebserkrankungen behandelt werden können.

Der Erfindung liegt die Erkenntnis zugrunde, dass eine solche Vakzine erhalten werden kann, wenn sie Antigen-Mimotope, welche mit dem HMW-MAA assoziiert sind oder deren funktionelle Varianten als wirksame Bestandteile enthält.

Mit der erfindungsgemäßen Vakzine ist eine aktive Immunisierung gegen Krebserkrankungen möglich, die mit dem High Molecular Weight Melanoma Associated Antigen (HMW-MAA) assoziiert sind. Somit kann eine Prophylaxe gegen diese Krebserkrankungen, bei welchen es sich meist um Melanome handelt, erhalten werden. Darüberhinaus kann die erfindungsgemäße Vakzine auch zur Therapie einer bereits bestehenden Krebserkrankung oder begleitend zu herkömmlichen Krebserkrankungen eingesetzt werden. Durch die Anwendung der erfindungsgemäßen Vakzine können die erheblichen Nachteile herkömmlicher Krebsbehandlungs-methoden wie etwa der Chemo- oder Strahlentherapie ganz oder teilweise vermieden werden.

Die erfindungsgemäße Vakzine besteht aus mindestens einem Peptid mit einer Aminosäuresequenz ausgewählt aus den folgenden Aminosäuresequenzen: TRLQAVKYP, TRTNPWPAL, TRTQPGRFP, TRTKAWPSP, CSLPYIARYAC, CGPRCTGPRCC und CQLPPSAQYAC. Die erfindungsgemäßen Peptide können auch mit anderen Peptiden oder Polypeptiden oder mit weiteren chemischen Gruppen wie Glycosylgruppen, Lipiden, Phosphaten, Acetylgruppen oder ähnlichem verknüpft sein, soweit sie deren Wirkung nicht nachteilig beinflussen.

In einer bevorzugten Ausführungsform wird das Peptid mit einem immunogenen Träger konjugiert. Solche Träger können Makromoleküle aller Art sein. Die Konjugation mit einem Träger hat zur Folge, dass die Immunogenität der Vakzine erhöht wird.

Bevorzugt wird das Peptid oder dessen funktionelle Variante an Keyhole Limpet Hemocyanin (KLH) oder Tetanustoxoid (TT) konjugiert.

Die Konjugation der Peptide an das Trägermaterial kann auf beliebige Weise erfolgen, beispielsweise auf gentechnologischem oder chemischem Weg, d. h. die Verknüpfung von Träger und funktioneller Gruppe erfolgt durch eine chemische Reaktion. Auf gentechnologischem Wege kann die Kopplung des Proteinträgermoleküls mit dem Peptid so hergestellt werden, dass eine für die Gesamtsequenz des Konjugates kodierende DNA- oder RNA-Sequenz in ein Expressionssystem eingebaut wird, von dem das Gesamtkonjugat dann exprimiert wird. Diese Form der Konjugation kann selbstverständlich nur für den Fall angewendet werden, dass auch das Gesamtkonjugat ein Proteinmolekül ist.

Bevorzugterweise werden die Peptide auf chemischem Wege mit dem Träger konjugiert. Das heißt, die Verknüpfung von Peptid und dem Träger zum Konjugat erfolgt auf chemischem Wege.

Die Peptide können als mono-, di-, tri- oder Oligomer mit dem Träger konjugiert werden. Solche Konjugationen sind beispielsweise in der Druckschrift von Th. H. Turpen, F. J. Reinel, Y. Charoenvit, S. L. Hoffmann, V. Fallarme in Bio/Technology 1995, Band 13, Seiten 53 - 57 am Beispiel der Konjugation von Epitopen mit makromolekularen Trägern beschrieben. Auf den Offenbarungsgehalt dieser Druckschrift wird hiermit Bezug genommen. Die beschriebenen Vorgehensweisen lassen sich analog auf die Herstellung der Konjugate für die erfindungsgemäße Vakzine übertragen.

Wird die Konjugation eines di- oder oligomeren Peptidkonjugats auf dem Weg des oben beschriebenen gentechnologischen Verfahrens durchgeführt, so werden die für die Peptide kodierenden DNA- oder RNA-Abschnitte ein- oder mehrmals hintereinander gereiht in die für den Träger kodierende DNA- oder RNA-Sequenz integriert. Dadurch wir die Expression di- oder oligomerer Peptidkonjugate erreicht.

Die Mono- oder Oligomeren der Peptide können sowohl in einfacher als auch in multipler Form an den Träger konjugiert werden, d. h. an einen Träger werden ein oder mehrere Peptidmoleküle angehängt.

Die erfindungsgemäße Vakzine kann auf verschiedene Arten appliziert werden. Die Verabreichung der die Peptide selbst enthaltenden Vakzine kann beispielsweise intravenös, subkutan oder auch durch orale Einnahme der Vakzine in Kapsel- oder Tablettenform erfolgen. Enthält die Vakzine funktionelle Nukleinsäurevarianten der Peptide, kann die Verabreichung auch mit Hilfe einer ex-vivo Prozedur erfolgen, die die Entnahme von Zellen aus einem Organismus, das Eindringen der erfindungsgemäßen Vakzine in diese Zellen, und das Wiedereindringen der behandelten Zellen in den Organismus umfasst.

Die erfindungsgemäße Vakzine kann in vielfältiger Weise auf gentechnologischem oder chemischen Weg hergestellt werden. Handelt es sich um einen chemischen Weg, so bietet sich die Festphasen-peptidsynthese an.

Ein Beispiel für ein gentechnologisches Herstellungsverfahren ist die Manipulation von Mikroorganismen wie E. Coli. Diese werden so manipuliert, dass sie die Peptide als solche oder die Gesamtkonjugate bestehend aus Peptid und daran gekoppelten Träger exprimieren.

Bevorzugterweise werden die Peptide auf chemischen Wege synthetisch dargestellt. In einer bevorzugten Ausführungsform geschieht dies mit Hilfe der Festphasensynthese. Weiter bevorzugt wird das synthetisch hergestellte Peptid auf chemischem Weg mit einem Träger wie KLH oder TT verknüpft.

Die erfindungsgemäße Vakzine kann zur prophylaktischen und akuten Behandlung von Menschen und Tieren, die mit dem High Molecular Weight Melanoma Associated Antigen (HMW-MAA) assoziierte Krebsarten entwickeln können, eingesetzt werden.

Gegenstand der vorliegenden Offenbarung ist weiterhin ein Antigen-Mimotop der extrazellulären Domäne des High Molecular Weight Melanoma Associated Antigen (HMW-MAA), welches dadurch gekennzeichnet ist, dass es immunologisch durch den monoklonalen HMW-MAA Antikörper 225.28S erkannt wird und mindestens ein Peptid einer Aminosäuresequenz mit einer Länge von 6 - 14 Aminosäuren umfasst.

Das erfindungsgemäße Antigen-Mimotop kann zum einen einen wesentlichen Bestandteil der oben beschriebenen Vakzine darstellen, zum anderen jedoch ist es auch dazu geeignet, die erhaltene Immunantwort bei einem bereits vakzinierten Patienten zu überprüfen. Es kann somit sowohl als Vakzinbestandteil, als auch als diagnostisches Mittel in vitro zur Überprüfung eines Impferfolges angewandt werden

Das beschriebene Antigen-Mimotop umfasst mindestens ein Peptid mit einer Länge von 8-12 Aminosäuren.

Bevorzugt umfasst das Antigen-Mimotop mindestens ein Peptid mit einer Länge von 9 - 11 Aminosäuren.

In der erfindungsgemäßen Ausführungsform besteht das Antigen-Mimotop aus mindestens einem Peptid mit einer Aminosäuresequenz, ausgewählt aus den folgenden Sequenzen: TRLQAVKYP, TRTNPWPAL, TRTQPGRFP, TRTKAWPSP, CSLPYIARYAC, CGPRCTGPRCC und CQLPPSAQYAC.

Zum Auffinden der Aminosäuresequenzen wird ein Verfahren angewandt, bei dem Phagenbibliotheken, welche Peptide einer bestimmten Sequenzlänge präsentieren, in unterschiedlicher Stärke an den HMW-MAA Antikörper 225.28S gebunden werden. Die Phagenbibliotheken repräsentieren dabei die unterschiedlichsten Sequenzzusammensetzungen einer bestimmten Peptidlänge und werden bei diesem Panning dahingehend ausgewählt, dass nur diejenigen Peptidsequenzen, welche die höchste Affinität zum Antikörper aufweisen, selektiert werden. Nach mehrfacher Wiederholung dieses Prozesses mit den jeweils selektierten Peptiden ist es möglich, solche Sequenzen mit der höchsten Affinität zum Antikörper zu isolieren. Die Identifizierung der entsprechenden Aminosäuresequenz erfolgt über herkömmliche gentechnologische Methoden. Die aufgefundenen Mimotopsequenzen müssen nicht notwendigerweise eine Sequenzhomologie zur extrazellulären Domäne des HMW-MAA aufweisen. Vielmehr genügt es, dass sie in der Lage sind, aufgrund ihrer strukturellen Eigenschaften an das Paratop des HMW-MAA Antikörpers 225.28S zu binden.

Die im einzelnen genannten Peptidsequenzen können insofern variieren, sofern einzelne Ersetzungen, Additionen und/oder das Weglassen einer oder mehrerer Aminosäuren die Funktion des Peptides, d. h. seine Fähigkeit an das Paratop des Antikörpers 225.28S zu binden, nicht stark beeinträchtigt. Die erfindungsgemäßen Peptide können auch mit anderen Peptiden oder Polypeptiden oder mit weiteren chemischen Gruppen wie Glykosylgruppen, Lipiden, Phosphaten, Acetylgruppen oder ähnlichen verknüpft sein, soweit sie deren Wirkung nicht nachteilig beeinflussen.

Bevorzugt ist das Antigen-Mimotop, d.h. das Peptid oder dessen funktionelle Variante an einen immunogenen Träger konjugiert. Weiterhin bevorzugt werden als Träger Keyhol Limpet Hemocyanin (KLH) oder Tetanustoxoid (TT) verwendet. Es sind jedoch auch andere Träger wie etwa Bovine Serum Albumin (BSA) denkbar.

Wird das Antigen-Mimotop als diagnostisches Mittel verwendet, so wird es bevorzugt mit einem solchen immunogenen Träger konjugiert, welcher nicht zur vorausgehenden Vakzinierung verwendet wurde. Damit wird bei der Überprüfung des Impferfolges verhindert, dass das diagnostische Mittel auf Antikörper reagiert, die gegen den Träger-Anteil der Vakzine gebildet wurden und daher nicht zu einer Krankheitsprophylaxe bzw. zu einer Therapie dienen.

Analog zu der erfindungsgemäßen Vakzine können die Antigen-Mimotope sowohl auf chemischem als auch auf gentechnologischen Wege hergestellt werden. Weiterhin ist es möglich, das Antigen-Mimotop als Monomeres, Dimeres, Trimeres usw. an den Träger zu koppeln. Außerdem kann das Antigen-Mimotop einfach oder mehrfach an den Träger gebunden werden.

## Patentansprüche

1. , Vakzine gegen Krebserkrankungen, die mit dem High Molecular Weight Melanoma Associated Antigen (HMW-MAA) assoziiert sind, **dadurch gekennzeichnet, dass** sie immunologisch durch den monoklonalen HMW-MAA Antikörper 225.28S erkannt wird und aus einem Peptid mit einer Aminosäuresequenz, ausgewählt aus den Aminosäuresequenzen:
TRLQAVKYP,
TRTNPWPAL,
TRTQPGRFP,
TRTKAWPSP,
CSLPYIARYAC,
CGPRCTGPRCC und
CQLPPSAQYAC. besteht.

2. Vakzine nach Anspruch 1, **dadurch gekennzeichnet, dass** das Peptid mit einem immunogenen Träger konjugiert ist.

3. Vakzine nach Anspruch 2, **dadurch gekennzeichnet, dass** als Träger Keyhole Limpet Hemocyanin (KLH) oder Tetanustoxoid (TT) verwendet wird.

4. Vakzin nach Anspruch 2 oder 3, **dadurch gekennzeichnet, dass** die Konjugation an den Träger chemisch erfolgt.

5. Vakzine nach einem der Ansprüche 2 bis 4, **dadurch gekennzeichnet, dass** das Peptid als Monomeres, Dimeres, Trimeres oder Oligomer an den Träger gekoppelt ist.

6. Vakzine nach einem der Ansprüche 2 bis 5, **dadurch gekennzeichnet, dass** das Peptid einfach oder mehrfach an den Träger konjugiert ist.

7. Antigen-Mimotop der extrazellulären Domäne des High Molecular Weight Melanoma Associated Antigen (HMW-MAA) als diagnostisches Mittel, **dadurch gekennzeichnet dass** es immunologisch durch den monoklonalen HMW-MAA Antikörper 225.28S erkannt wird und aus einem Peptid mit einer Aminosäuresequenz, ausgewählt aus den Sequenzen:
TRLQAVKYP,
TRTNPWPAL,
TRTQPGRFP,
TRTKAWPSP,
CSLPYIARYAC,
CGPRCTGPRCC und
CQLPPSAQYAC, besteht.

8. Antigen-Mimotop nach Anspruch 7, **dadurch gekennzeichnet, dass** das Peptid mit einem immunogenen Träger konjugiert ist.

9. Antigen-Mimotop nach Anspruch 8, **dadurch gekennzeichnet, dass** als Träger Keyhole Limpet Hemocyanin (KLH) oder Tetanustoxoid (TT) verwendet wird.

## Claims

1. Vaccine against cancers that are associated with the high molecular weight melanoma associated antigen (HMW-MAA), **characterized in that** it is immunologically recognized by the monoclonal HMW-MAA antibody 225.28S and consists of a peptide with an amino acid sequence selected from the amino acid sequences:
TRLQAVKYP,
TRTNPWPAL,
TRTQPGRFP,
TRTKAWPSP,
CSLPYIARYAC,
CGPRCTGPRCC and
CQLPPSAQYAC.

2. Vaccine according to claim 1, **characterized in that** the peptide is conjugated with an immunogenic carrier.

3. Vaccine according to claim 2, **characterized in that** keyhole-limpet hemocyanin (KLH) or tetanus toxoid (TT) is used as carrier.

4. Vaccine according to claim 2 or 3, **characterized in that** the conjugation with the carriers is chemical.

5. Vaccine according to one of claims 2 to 4, **characterized in that** the peptide is coupled to the carrier as monomer, dimer, trimer or oligomer.

6. Vaccine according to one of claims 2 to 5, **characterized in that** the peptide is mono- or polyconjugated with the carrier.

7. Antigen mimotope of the extracellular domain of the high molecular weight melanoma associated antigen (HMW-MAA) as diagnostic agent, **characterized in that** it is immunologically recognized by the monoclonal HMW-MAA antibody 225.28S and consists of a peptide with an amino acid sequence selected from the amino acid sequences:
TRLQAVKYP,
TRTNPWPAL,
TRTQPGRFP,
TRTKAWPSP,
CSLPYIARYAC,
CGPRCTGPRCC and
CQLPPSAQYAC.

8. Antigen mimotope according to claim 7, **characterized in that** the peptide is conjugated with an immunogenic carrier.

9. Antigen mimotope according to claim 8, **characterized in that** keyhole-limpet hemocyanin (KLH) or tetanus toxoid (TT) is used as carrier.

## Revendications

1. Vaccin contre des maladies cancéreuses qui sont associées à l'antigène associé au mélanome de haut poids moléculaire (HMW-MAA), **caractérisé en ce qu'**il est reconnu immunologiquement par l'anticorps monoclonal 225-28S de HMW-MAA, et consiste en un peptide de séquences peptidique, choisie parmi les séquences peptidiques :
TRLQAVKYP,
TRTNPWPAL,
TRTQPGRFP,
TRTKAWPSP,
CSLPYIARYAC,
CGPRCTGPRCC et
CQLPPSAQYAC.

2. Vaccin selon la revendication 1, **caractérisé en ce que** le peptide est conjugué avec un support immunogène.

3. Vaccin selon la revendication 2, **caractérisé en ce qu'**on utilise comme support l'anatoxine tétanique (TT) ou l'hémocyanine de patelle (KLH).

4. Vaccin selon la revendication 2 ou 3, **caractérisé en ce que** la conjugaison au support est réalisé chimiquement.

5. Vaccin selon l'une quelconque des revendications 2 à 4, **caractérisé en ce que** le peptide est couplé au support comme monomère, dimère, trimère ou oligomère.

6. Vaccin selon l'une quelconque des revendications 2 à 5, **caractérisé en ce que** le peptide est mono conjugué ou multi conjugué au support.

7. Mimotope d'antigène du domaine extracellulaire de l'antigène associé au mélanome de haut poids moléculaire (HMW-MAA) comme moyen diagnostique, **caractérisé en ce qu'**il est reconnu immunologiquement par l'anticorps monoclonal 225.28S de HMW-MAA, et consiste en un peptide de séquence peptidique choisie parmi les séquences :
TRLQAVKYP,
TRTNPWPAL,
TRTQPGRFP,
TRTKAWPSP,
CSLPYIARYAC,
CGPRCTGPRCC et
CQLPPSAQYAC.

8. Mimotope d'antigène selon la revendication 7, **caractérisé en ce que** le peptide est conjugué avec un support immunogène.

9. Mimotope d'antigène selon la revendication 8, **caractérisé en ce qu'**on utilise comme support l'anatoxine tétanique (TT) ou l'hémocyanine de patelle (KLH).


### SEQUENZPROTOKOLL

<110> BioLife Science Forschungs und Entwicklungsges.mbH
<120> Antigen-Mimotope und Vakzine gegen Krebserkrankungen
<130> K 37 493/1yv
<140>
   <141>
<160> 7
<170> Patientin Ver. 2.1
<210> 1
   <211> 9
   <212> PRT
   <213> Künstliche Sequenz
<220>
   <223> Beschreibung der künstlichen Sequenz:
   Anigen-Mimotop des High Molecular Weight Melanoma
   Associated Antigen (HMW-MAA)
<400> 1
   Thr Arg Leu Gln Ala Val Lys Tyr Pro
   1 5
<210> 2
   <211> 9
   <212> PRT
   <213> Künstliche Sequenz
<220>
   <223> Beschreibung der künstlichen Sequenz:
   Anigen-Mimotop des High Molecular Weight Melanoma
   Associated Antigen (HMW-MAA)
<400> 2
   Thr Arg Thr Asn Pro Trp Pro Ala Leu
   1 5
<210> 3
   <211> 9
   <212> PRT
   <213> Künstliche Sequenz
<220>
   <223> Beschreibung der künstlichen Sequenz:
   Anigen-Mimotop des High Molecular Weight Melanoma
   Associated Antigen (HMW-MAA)
<400> 3
   Thr Arg Thr Gln Pro Gly Arg Phe Pro
   1 5
<210> 4
   <211> 9
   <212> PRT
   <213> Künstliche Sequenz
<220>
   <223> Beschreibung der künstlichen Sequenz:
   Anigen-Mimotop des High Molecular Weight Melanoma
   Associated Antigen (HMW-MAA)
<400> 4
   Thr Arg Thr Lys Ala Trp Pro Ser Pro
   1 5
<210> 5
   <211> 11
   <212> PRT
   <213> Künstliche Sequenz
<220>
   <223> Beschreibung der künstlichen Sequenz:
   Anigen-Mimotop des High Molecular Weight Melanoma
   Associated Antigen (HMW-MAA)
<400> 5
   Cys Ser Leu Pro Tyr Ile Ala Arg Tyr Ala Cys
   1 5 10
<210> 6
   <211> 11
   <212> PRT
   <213> Künstliche Sequenz
<220>
   <223> Beschreibung der künstlichen Sequenz:
   Anigen-Mimotop des High Molecular Weight Melanoma
   Associated Antigen (HMW-MAA)
<400> 6
   Cys Gly Pro Arg Cys Thr Gly Pro Arg Cys Cys
   1 5 10
<210> 7
   <211> 11
   <212> PRT
   <213> Künstliche Sequenz
<220>
   <223> Beschreibung der künstlichen Sequenz:
   Anigen-Mimotop des High Molecular Weight Melanoma
   Associated Antigen (HMW-MAA)
<400> 7
   Cys Gln Leu Pro Pro Ser Ala Gln Tyr Ala Cys
   1 5 10